# EUROPEAN PATENT SPECIFICATION

(11) **EP 3 261 726 B1**
(45) Date of publication and mention of the grant of the patent: **17.04.2019**
(21) Application number: 16706531.7
(22) Date of filing: 23.02.2016
(51) Int. Cl.: A61Q 19/00, A61K 8/9789, A61K 8/34, A61K 8/365, A61K 36/76, A61K 8/60, A61Q 19/10, A61Q 17/04, A61K 8/04, A61K 31/047, A61K 9/00

(54) **COMPOSITION FOR THE TREATMENT OF ACNE**
ZUSAMMENSETZUNG ZUR BEHANDLUNG VON AKNE
COMPOSITION POUR LE TRAITEMENT DE L'ACNÉ

(30) Priority: 27.02.2015 EP 15000576
(43) Date of publication of application: 03.01.2018
(60) Divisional application: 19161351.2
(73) Proprietor: Rottapharm Ltd., Dublin 15 (IE)
(72) Inventor: GASPARRI, Franco, 40020 Casalfiumanes (BO) (IT); MANTEGAZZA, Raffaella, 20800 Monza (MB) (IT); BELTRANDI, Martina, 12084 Mondovi (IT); ZARNARDI, Andrea, 20156 Milano (IT)
(74) Representative: FRKelly
(86) International application number: PCT/EP2016/000312
(87) International publication number: WO 2016/134846

(56) References cited:
- WO-A1-2008/046791
- DE-A1- 10 341 179
- DE-A1-102012 223 401
- US-A1- 2010 172 847
- DATABASE GNPD [Online] MINTEL; 1 December 2010 (2010-12-01), Boscia: "Clear Complexion Moisturizer with Botanical Blast", XP002743241, Database accession no. 1448967
- DATABASE GNPD [Online] MINTEL; 1 November 2011 (2011-11-01), Kate Somerville: "Acne 24 Hour Pimple Punisher", XP002743242, Database accession no. 1676345
- NN: "Salicylic acid", en.wikipedia.org, 26 July 2015 (2015-07-26), pages 1-9, XP002743243, Retrieved from the Internet: URL:https://en.wikipedia.org/wiki/Salicyli c_acid [retrieved on 2015-08-12]

## Description

The present invention describes a cosmetic or dermatological composition for use in the treatment of acne comprising a combination of vegetal extracts titrated in salicin and 1,2-alkanediols, preferably 1, 2-decanediol and willow bark (*Salix alba*) extract.

Acne vulgaris is the most frequently diagnosed dermatosis in patients in the age between 11 and 30. It is believed that acne affects about 80% of persons in this age group or even, taking into account lesions of low intensity, 100% of young people. The aetiopathogenesis of acne is multifactorial. In all patients with acne, the following symptoms occur: excessive sebum production, excessive keratosis of excretory ducts and openings of sebaceous glands, development of bacterial flora and release of inflammatory mediators in the skin.

In 95% of the patients, lesions are situated on the face and on the upper parts of the trunk, occasionally on the other parts of the body, and due to this location and the chronic nature of the disease, the patients have serious psychological problems.

Excessive sebum production and accumulation and blocked sebaceous gland openings favour bacterial colonization. Microcomedones are inhabited mostly by *Propionibacterium acnes,* a micro-organism mainly involved in the development of acne. Other bacteria are inter alia: *Staphylococcus epidermidis* and *Malasezzia furfur.* Due to the presence of lipase, this microorganism hydrolyses sebum di- and triglycerides to free fatty acids. Free fatty acids, which arise during the hydrolysis process, have the irritating, proinflammatory effect and intensify follicular keratosis. Also hyaluronidase, proteases and neuraminidases, produced by *Propionibacterium acnes,* have the pro-inflammatory effect. Moreover, this microorganism releases low-molecular chemotactic factors (peptides), attracting neutrocytes, and it activates both the alternative complement pathway and a classic immune response. The activator of the alternative complement pathway is the cell wall of *Propionibacterium* (B. Bergler-Czop, International Journal of Cosmetic Science, 2014, 36, 187-194).

Microorganisms like *Propionibacterium acnes* can prompt the secretion of a variety of cutaneous anti-microbial peptides (AMPs). The inflammatory effects of *P. acnes* do not require the organism to be alive or be present in its entirety. In vitro, supernatants containing either membranous peptidoglycans (PG) and lipopolysaccharides (LPS) or, cytosolic contents or membrane proteins were able to increase keratinocyte expression of TLR2 and TLR4.

It is well known that further cytokines play a significant role in acne inflammation. *Propionibacterium acnes* triggers the pro-inflammatory mediators through activation of Toll-like receptor 2 (TLR2). Among these mediators is IL-8 the expression of which is induced by *P. acnes.*

Tumor necrosis factor-alpha (TNF-alpha) is another pro-inflammatory cytokine involved in acne pathogenesis (Archives of Dermatological Research 2008, Volume 300, No 7, pp 371-376).

The extract of white willow bark *(Salix alba)* contains salicin and its derivatives, which have been used for longer than a century as antipyretic agents and in the treatment of many rheumatic disorders.

Herbal medicinal products such as various formulations from the bark of Salix species are well recognised throughout Europe for treating inflammatory conditions and pains of different origin.

*Salix* extracts have demonstrated analgesic, antiphlogistic and antipyretic effects in various animal models (Loniewski et al. 2002).

For example, dose-dependent effects have been reported in the hot-plate and carrageenan-induced paw edema tests in rats (established test procedures to demonstrate analgesic and anti-inflammatory effects, respectively) for a *Salix* extract containing 12% salicin (Loniewski et al. 2002).

Salicin has been known as a potent anti-inflammatory agent when taken orally.

It is believed that salicin may have anti-aging capabilities when applied topically to human skin. The data from a study show that topical application of a serum formulation containing 0.5% salicin offers wide-spectrum anti-aging benefits, targeting the major signs of visible human skin aging. Statistical improvements were indicated in structure-related signs of skin aging against baseline. These include wrinkles, fine lines, jaw-line contour, firmness, extensibility, and density. Statistical improvements were also seen in mottled pigmentation, uneven skin tone, radiance, hydration and tactile roughness against baseline. These results substantiate salicin's ability to improve the visible signs of aging, when applied topically to human skin in a product formulation. (Gopaul Remona et al., An evaluation of the effect of a topical product containing salicin on the visible signs of human skin aging Journal of Cosmetic Dermatology, 9, 196-201)

From prior art it has been known that salicin has may be useful as anti-irritative active compound in cosmetic and topical dermatological preparations (EP 0801946).

Salicin is also useful in combinations with additional herbal extracts. DE 10034328 discloses a cosmetic formulation for external application to skin, especially skin reddened by acne rosaceae, containing quercetin, rutin, salicin and escin in aqueous medium.

1,2-Alkanediols like 1,2-pentanediol (INCI: Pentylene Glycol), 1,2-hexane diol (INCI: 1,2-Hexanediol) and 1,2-octanediol (INCI: Caprylyl Glycol) as well as combinations thereof are multifunctional ingredients widely used in personal care products as moisturizers and antibacterial agents. It is well known to the person skilled in the art that these substances show specific antibacterial activity against *P. acnes.*

The commercial product "Boscia Clear Complexion" (Mintel Record ID: 1448967, published Dec 2010) discloses a treatment moisturizer comprising Salix alba Bark Extract and also Decylene Glycol.

WO 2011117126 describes a combination of 1,2-decanediol and licochalcone A and carnitine which is effective against *Propionibacterium acnes* (*P. acnes*) without irritating the skin.
EP 1731036 discloses mixtures containing different 1,2-alkanediols for acne treatment. Conventional products for the treatment of oily and blemished skin, eg. aqueous/ethanolic and / or surfactant-containing cleaning products have the disadvantage to strain usually the skin to dry out and work only little skin caring. It is particularly disadvantageous that the body's reaction to the use of ethanol and / or detergents-containing solutions over a longer period of time may be an overproduction of sebum, which counteracts the primary therapeutic goal of sebum reduction in oily, blemished and acne prone skin.
For the topical treatment of acne there are acne agents, such as strong oxidizing agents, such as. benzoyl peroxide; alpha-hydroxy acids, such as salicylic acid and lactic acid; aliphatic dicarboxylic acids such as azelaic acid; retinoids such as tretionoin (synonym: all-trans-retinoic acid), all-trans-retinal and 13-cis-retinoic acid (isotretinoin); antiandrogens (5alpha-reductase) and antibiotics such as clindamycin, tetracycline and erythromycin.
Said substances usually have only a very moderate antimicrobial activity against *P. acnes* and have to be used in a relatively high concentration - benzoyl peroxide for example, with up to 5 wt .-%, and azelaic acid with up to 20 wt .-% - in cosmetic and dermatological formulations.
Due to the high dosage, however, the skin is extremely disadvantageous heavily overused, which is manifested particularly in very dry skin, which is often associated in part with severe skin irritation due to the strong reduction of the pH of the skin.

The effectiveness of the strong oxidizing agent such as benzoyl peroxide, the alpha hydroxy acids such as salicylic acid and lactic acid, and the aliphatic dicarboxylic acids such as azelaic acid is based on the inhibition of *P. acnes.*

It is desirable to have a cosmetic or dermatological preparation to provide acne treatment that has an improved activity against *Propionibacterium acnes* (*P. acnes*) and improved inflammatory activity especially against inflamed comedones without severely irritating the skin.

The problem is solved by a cosmetic or dermatological composition comprising a combination of 1, 2-decanediol and willow bark extract. This preparation surprisingly shows a synergistic antibacterial and anti-inflammatory effect compared to the single substances.

### Experimental Part

### Determination of MIC and MBC on Gram positive bacteria

### Aim of the test

The aim of the test was to determine the antimicrobial activity of one or more samples against a micro-organism. The values obtained must be considered characteristic of the test sample for a determined microbial species under determined test conditions.

### Description of the method

A series of test tubes was prepared containing the culture broth inoculated with the test micro-organism and the substance to be tested was introduced at decreasing scalar concentrations.

The cultures were incubated at the optimal temperature and under optimal growth conditions of the preselected micro-organism and for a sufficient period of time to demonstrate development.

The test tubes in which the microbial population developed were then determined by observing visually the turbidity that is indicative of growth. Test tubes in which the substance is present at a sufficient concentration to inhibit growth remain clear. When the nature itself of the sample prevents visual reading or if it is wished to determine whether the lack of development is due to a microbiostatic or microbicidal action, transfers are made to appropriate agar media for confirmation.

### Criteria of interpretation

The concentration of sample at which growth of the micro-organisms is inhibited is considered to be the Minimum Inhibitory Concentration (MIC), while that at which viable micro-organisms are no longer present is considered to be the Minimum Bactericidal Concentration (MBC)

### A) Evaluation of the antibacterial activity of different substances against Staphylococcus species

A test to evaluate antibacterial activity against Gram positive bacteria was conducted evaluating the following substances:
Bakuchiol, Lactoferrin, Lauric acid, Resveratrol, *Salix alba* extract.

The antibacterial activity of these substances against *Staphylococcus aureus acnes* ATCC 25923 and *Staphylococcus epidermidis* ATCC 12228 was tested according to the above-described method. The results are shown in Table 1.

**Table 1**

| | *Staphylococcus aureus* ATCC 25923 | | *Staphylococcus epidermidis* ATCC 12228 | |
|---|---|---|---|---|
| | MIC (µg/ml) | MBC (µg/ml) | MIC (µg/ml) | MBC (µg/ml) |
| Bakuchiol | 6.25 | 100 | 6.25 | 100 |
| Lactoferrin | >20000 | - | >20000 | - |
| Lauric Acid | 250 | 1.000 | 500 | 1000 |
| Resveratrol | 1000 | > 100000 | 250 | > 100000 |
| *Salix alba* extract | 30000 | 100000 | 30000 | 100000 |

| | | | | |
|---|---|---|---|---|
| Minimum Inhibition Concentration (MIC) and Minimum Bactericide Concentration (MBC) of different substances against *Staphylococcus aureus acnes* and *Staphylococcus epidermidis* | | | | |

The test showed poor activity of *Salix alba* extract against Gram positive bacteria with respect to other substances tested.

### B) Evaluation of the antibacterial activity of different substances against P. acnes

In a further set of experiments we tested the antibacterial activity of *Salix alba* extract and 1,2-decanediol, a substance which is known to show antibacterial activity against *P. acnes*, alone and in combination according to the above-described method.

The microbial strain used was *Propionibacterium acnes* ATCC 11827 titrated at a concentration of 2.6 x 104 cells per milliliter of medium.

The result is shown in Table 2.

**Table 2**

| | *Propionibacterium acnes* | |
|---|---|---|
| | MIC (µg/ml) | MBC (µg/ml) |
| *Salix alba* extract | 1560 | 3120 |
| 1,2-decanediol | 620 | 1250 |
| *Salix alba* extract +1,2-decanediol (1:1) | 620 | 620 |
| benzoyl peroxide | 480 | 480 |

| | | |
|---|---|---|
| Minimum Inhibition Concentration (MIC) and Minimum Bactericide Concentration (MBC) of 1,2-Decanediol and *Salix alba* extract against *Propionibacterium acnes* | | |

*Salix alba* extract alone shows only poor antibacterial activity.

1,2-decanediol is known to be effective against *P. acnes.*

The combination (1:1) of *Salix alba* extract and 1,2-decanediol surprisingly displayed a synergistic antibacterial effect against *P. acnes.*

A combination of 1,2-decanediol and *Salix alba* extract is capable to obtain the same MIC of the double concentration of 1,2-decanediol and to improve dramatically its MBC.

The selected order degree of the association is in line with benzoyl peroxide that is commonly known as strong antibacterial agent against *P. acnes.*

### In vitro evaluation of the anti-inflammatory activity of Salix alba extract and 1,2-decanediol

The aim of the test was the evaluation of the anti-inflammatory activity of *Salix alba* extract, 1,2-decanediol and their combination by means of the study of TNFalpha, IL1alpha, IL8 synthesis modulation in a biological experimental model (keratinocyte cell culture HaCat).

TNFalpha, IL1alpha, IL8 are inflammatory mediators related to the acneic process.

Keratinocyte cell culture were selected as experimental model on the base that the acneic process is performed inside the sebaceous glands follicle, a tissue composed by keratinocytes.

In order to stimulate the inflammatory process in our experimental model, lipopolysaccharides (LPS) were selected. LPS are pro-inflammatory mediators secreted by bacteria, e.g. by *P. acnes.*

1,2-decanediol (SMAL) was emulsified with corn oil (37°C) in mechanical agitation and addition of culture medium, starting from the following ratio: 0.05 g emulsified with 100 µl corn oil in a final volume of culture medium of 1 ml (37°C).

*Salix alba* extract (WMAL) was direct diluted in culture medium, starting from the following ratio: 0.05 g in 1 ml (37°C); subsequent dilutions in culture medium (37°C).

For the test execution, human keratinocytes (HaCat) were treated with LPS (Lipopolysaccharide from *Escherichia coli,* 10 ng/ml) and with tested product at 3 concentrations, chosen among non-cytotoxic ones after a preliminary cytotoxicity test.

At the end of the monitored experimental period, cytokine levels were measured by means of ELISA assay.

Results were compared to negative control (cells not subjected to LPS, CTR-) and positive control (cells treated with LPS, CTR+).

A preliminary cytotoxicity test was performed on the two products by using a concentration range from 5.0% to 0.0012% (serial dilution 1:2). According to obtained results, the following concentrations were chosen for the analysis of the antinflammatory activity:
1,2-decanediol (SMAL): 0.002%
*Salix alba* extract (WMAL): 0.04% - 0.02% - 0.01%

A second cytotoxicity test was performed on the mix of the two raw materials preliminary to the beginning of the test to verify the action synergism in the antinflammatory activity; tested concentrations were the following:
SMAL 0.002% + WMAL: 0.04% - 0.02% - 0.01%

Culture media of controls and cells treated with tested product were used for the dosage of pro-inflammatory cytokines TNFalpha, IL1 alpha and IL8 by means of ELISA method.

Commercial ELISA kits were used for the determination of cytokine quantity.

The quantitative determination uses a calibration curve made-up of standard known and growing concentrations of standard cytokine.

The data obtained in the different experimental groups were subjected to statistical analysis and compared according to t-test. The variations are considered significant for p<0.05.

In table 3 below the data obtained for each tested series are reported.

The results are expressed as cytokine released into the medium during the experimental period (mean value ± dev.st.) and as mean % variation compared to the controls.

In order to compare the two different work sessions, the obtained data were normalized in a 0 - 100% scale, in which CTR- is 0 and CTR+ is 100%; all the other data were calculated in this interval.

On these data, statistical analysis by t-test was performed and variations are considered significant for p<0.05.

On the base of the results obtained, it is observed that SMAL and WMAL are able to significantly reduce the LPS-induced release of the inflammation markers TNFalpha, IL1 alpha and IL8, which are all related to acne.

For all substances analyzed, the substances WMAL and SMAL in combination show a synergistic effect.

**Table 3**

| TNF-ALPHA | Normalized data 0-100% | | | | |
|---|---|---|---|---|---|
| | | dev std | T-test vs CTR+ | T-test vs CTR+ +WMAL | T-test vs CTR+ +SMAL |
| CTR- | 0 | | | | |
| CTR+ | 100.00% | 6.2% | | | |
| WMAL 0,4% | 32.94% | 22.2% | 0.0545 | | |
| SMAL 0,02% | 18.45% | 3.9% | 0.0040 | | |
| WMAL 0,4%+SMAL 0,02% | -25.00% | 8.5% | 0.0042 | 0.0751 | 0.0224 |
| | | | | | |

| IL-8 | Normalized data 0-100% | | | | |
|---|---|---|---|---|---|
| | | dev std | T-test vs CTR+ | T-test vs CTR+ .+WMAL | T-test vs CTR+ +SMAL |
| CTR- | 0 | | | | |
| CTR+ | 100.00% | 9.4% | | | |
| WMAL 0,4% | 17.77% | 0.4% | 0.0064 | | |
| SMAL 0,02% | 34.56% | 0.3% | 0.0101 | | |
| WMAL 0,4%+SMAL 0,02% | -13.76% | 2.9% | 0.0011 | 0.0044 | 0.0019 |
| | | | | | |

| IL-6 | Normalized data 0-100% | | | | |
|---|---|---|---|---|---|
| | | dev std | T-test vs CTR+ | T-test vs CTR+ +WMAL | T-test vs CTR+ +SMAL |
| CTR- | 0 | | | | |
| CTR+ | 100.00% | 45.6% | | | |
| WMAL 0,4% | 75.81% | 111.8% | 0.8035 | | |
| SMAL 0,02% | 61.69% | 106.6% | 0.6864 | | |
| WMAL 0,4%+SMAL 0,02% | -21.43% | 7.2% | 0.0017 | 0.3444 | 0.3861 |

Dosage of cytokine in cell culture CTR-, CTR+, treated with SMAL, WMAL and their associations. The results are expressed as mean value ± dev.st. (expressed in ng/L) and as mean % variation compared to the controls.

Formulations can be of various nature both water-based, hydro-alcohol and anhydrous. Among the most commonly used include: surfactants systems (including even such as shampoo , bubble bath, shower gel, liquid soaps and solid soaps, micellar waters, liquid detergents), water -in-oil emulsions, oil-in-water emulsions, serums, gels, oils, aqueous solutions, hydroalcoholic solutions , anhydrous sticks, compact founding powders, lotions, milks, suncreams, after sun products and camouflage products (i.e. CC creams, BB creams).

It is optionally possible and advantageous to use the compositions according to the invention as a base for pharmaceutical formulations.

It is of course known to the person skilled in the art that cosmetic compositions are usually inconceivable without the customary auxiliaries and additives. Among these are included, for example, consistency-imparting agents, fillers, perfume, colorants, emulsifiers, additional active compounds such as vitamins or proteins, sunscreens, stabilisers, insect repellents, alcohol, water, salts, substances having proteolytic or keratolytic activity, thickeners, emollients, fatty acids, chelating agents, soothing agents, glycols, colorants, buffering substances.

The willow bark extract is prepared according to the Ph.Eur. monograph "Extracta". Whole or fragmented dried bark of young branches or whole dried pieces of current year twigs of *Salix* are extracted with water and/or ethanol. The total content of salicylic derivatives expressed in salicin has to be at least 5%; preferably between 10 and 20%.

The *Salix alba* extract according to the present invention has to be titrated to a total concentration of salicylic derivatives expressed as salicin not less than 20 % (w/w).

The ratio between *Salix alba* extract and 1,2-decanediol is between 1:100 and 100:1, or between 1:50 and 50:1, or between 1:10 and 10:1, preferably 1:5 and 5:1; more preferably 1:1.

The overall amount of *Salix alba* extract in the formula is in a range between 0.001% and 10%, preferably between 0.01 % (w/w) and 5 % (w/w), more preferably between 0.3 % (w/w) and 1 % (w/w).

The overall amount of 1,2-decanediol in the formula is in a range between 0.001 % (w/w) and 10 % (w/w), preferably between 0.01 % (w/w) and 5 % (w/w), more preferably between 0.3 % (w/w) and 1 % (w/w).

### Examples

The following examples shall not represent a restriction of the invention.

### Example 1

**Suncream**

| **INCI NAME** | **% (w/w)** |
|---|---|
| | |
| Avobenzone | 0-5 |
| Glycerol | 0-5,00 |
| Panthenol | 0-1,00 |
| Disodium EDTA | 0,10 |
| Glyceryl Stearate, PEG-100 Stearate (50%:50%) (Arlacel 165V P) | 1-5,00 |
| 4-Methylbenzylidene Camphor | 1-4,00 |
| Citrus Aurantius Amara | 0-1 |
| Xanthan Gum | 0-1 |
| Ascorbyl Tetraisopalmitate | 0,20 |
| Polyacrylamide (45%), C13-14 Isoparaffin (25%), Laureth-7 (8%), Aqua (22%) (Sepigel 305) | 0-2,00 |
| Methylene Bis-Benzotriazolyl Tetramethylbutylphenol, Aqua, Decyl Glucoside, Propylene Glycol, Xanthan Gum (Tinosorb A2B) | 0-2,00 |
| Ethylhexyl Methoxycinnamate | 0-8,00 |
| Niacinamide | 0-1 |
| Magnesium Aluminum Silicate | 0-0,50 |
| Dicaprylyl Carbonate | 1-5,00 |
| 1,2-Hexanediol, Caprylyl Glycol (50%:50%) (Symdiol 68) | 0,50 |
| Soy Isoflavones | 0,30 |
| Ethylhexyl Triazone | 0-2,00 |
| Titanium Dioxide (75%),Silica (10%), Dimethicone (15%) (Parsol TX) | 0-5 |
| 1,2-Decanediol | 0,30 |
| *Salix alba* (Willow) Bark Extract | 0,30 |
| Tocopheryl Acetate | 0,50 |
| Butylated hydroxytoluene | 0-0,05 |
| Hydroxyacetophenone | 0-0,50 |
| Demin. H₂O | ad 100,00 |

### Example 2

**Serum**

| **INCI NAME** | **% (w/w)** |
|---|---|
| | |
| Sodium Hydroxide | 0-8 |
| Glycolic Acid | 0-20,00 |
| Lactic Acid | 0-3 |
| Niacinamide | 1,00 |
| Caprylyl Glycol | 0-5,00 |
| Xanthan Gum | 0,10 |
| Polyacrylate Crosspolymer-6 | 2,20 |
| Dicaprylyl Ether | 0-4,00 |
| Salicylic Acid | 0-2 |
| 1,2-Decanediol | 1,00 |
| Polyglyceryl-6 Stearate (85-98%), Polyglyceryl-6 Behenate (2-15%) (Tego Care PBS 6) | 0-4 |
| *Salix alba* (Willow) Bark Extract | 0,30 |
| Aluminum Starch Octenylsuccinate | 0-2,00 |
| Dipotassium Glycirrhyzinate | 0-2 |
| Sodium Phytate, Aqua, Alcohol | 0,10 |
| Demin. H₂O | ad 100,00 |

### Example 3

**Hydrating Cream**

| **INCI NAME** | **% (w/w)** |
|---|---|
| | |
| Disodium EDTA | 0,10 |
| Glycerol | 0-7,00 |
| Sodium Hyaluronate | 0-1 |
| Niacinamide | 1,00 |
| Caprylyl Glycol | 0-2,00 |
| Xanthan Gum | 0,10 |
| Polyacrylate Crosspolymer-6 | 0-3,00 |
| Dicaprylyl Ether | 0-5,00 |
| 1,2-Decanediol | 0,30 |
| Polyglyceryl-6 Stearate (85-98%), Polyglyceryl-6 Behenate (2-15%) (Tego Care PBS 6) | 2,50 |
| *Salix alba* (Willow) Bark Extract | 0,3 |
| Lactic Acid | 0-1 |
| Sodium Phytate (48-52%), Aqua (48-52%), Alcohol(1%) (Dermosoft PA 3) | 0,05 |
| Demin. H₂O | ad 100,00 |

### Example 4

**Gel**

| **INCI NAME** | **% (w/w)** |
|---|---|
| | |
| Disodium EDTA | 0,10 |
| Glycerol | 0-3,00 |
| Niacinamide | 1,00 |
| Caprylyl Glycol | 0-2,00 |
| Xanthan Gum | 0,20 |
| Polyacrylate Crosspolymer-6 | 0-3,0 |
| 1,2-Decanediol | 0,50 |
| *Salix alba* (Willow) Bark Extract | 0,50 |
| Lactic Acid | 0,50 |
| Sodium Phytate (48-52%), Aqua (48-52%), Alcohol(1%) (Dermosoft PA 3) | 0,10 |
| Demin. H₂O | ad 100,00 |

### Example 5

**Liquid detergent**

| **INCI NAME** | **% (w/w)** |
|---|---|
| | |
| Disodium EDTA | 0,10 |
| Glycerol | 0-4,00 |
| Hydroxypropyl Guar Hydroxypropyltrimonium Chloride (Jaguar C162) | 0-1,0 |
| Niacinamide | 0,30 |
| Methylpropanediol (80%), Caprylyl Glycol (15%), Phenylpropanol (5%) (Dermosoft OMP) | 0-5,0 |
| Xanthan Gum | 0,30 |
| Sodium Cocoamphoacetate | 0-10,0 |
| Disodium Laureth Sulfosuccinate | 0-10,0 |
| 1,2-Decanediol | 1,00 |
| PEG-40 Hydrogenated Castor Oil | 1,00 |
| *Salix alba* (Willow) Bark Extract | 0,01 |
| Lactic Acid | 1,00 |
| Demin. H₂O | ad 100,00 |

### Example 6

**CC Cream**

| **INCI NAME** | **% (w/w)** |
|---|---|
| | |
| Aqua | 56,00 |
| Glycerol | 2,00 |
| Niacinamide | 0,50 |
| *Salix alba* (Willow) Bark Extract | 0,50 |
| Magnesium Sulfate Heptahydrate | 0,50 |
| Methylpropanediol (80%), Caprylyl Glycol (15%), Phenylpropanol (5%) (Dermosoft OMP) | 2,00 |
| Cetyl Dimethicone | 2,00 |
| Octyldodecanol | 10,00 |
| Ethylhexyl Palmitate | 8,00 |
| Dimethicone | 1,50 |
| Hydrogenated Castor Oil | 1,50 |
| 1,2-Decanediol | 1,00 |
| Cetyl PEG PPG-10/1 Dimethicone (Abil EM 90) | 2,50 |
| Titanium dioxide | 5,00 |
| Tocopheryl Acetate | 1,00 |
| Mica (48-59%), Titanium Dioxide (41-52%) (Timica Terra White) | 4,00 |
| Mica (44-54%), Iron Oxides (36-50%), Titanium Dioxide (2-8%) (Timica Terra Brown) | 2,00 |
| Demin. H₂O | ad 100,00 |

### Example 7

**Moisturizing cream**

| **INCI NAME** | **% (w/w)** |
|---|---|
| Disodium EDTA | 0 - 0,10 |
| Glycerin | 0 - 6,00 |
| Niacinamide | 0 - 1,00 |
| Sodium Phytate | 0 - 0,05 |
| Sodium Hyaluronate | 0,2 - 0,9 |
| *Salix alba* (Willow) Bark Extract | 0,01 - 0,3 |
| Caprylyl Glycol | 0,5 - 1,5 |
| Xanthan Gum | 0 - 0,30 |
| Polyacrilate Cosspolymer-6 | 0 - 1,50 |
| Dicaprylyl Ether | 1 - 5,00 |
| 1,2-Decanediol | 0 - 0,9 |
| Polyglyceryl-6 Stearate (85-98%), Polyglyceryl-6 Behenate (2-15%) (Tego Care PBS 6) | 2,50 |
| Aluminum Starch Octenylsuccinate | 0-2,00 |
| Lactic Acid | 0-0,15 |
| Demin. H₂O | ad 100 |

### Example 8

**Detergent**

| **INCI NAME** | **% (w/w)** |
|---|---|
| Glycerol | 0,1-2,00 |
| Disodium EDTA | 0-0,10 |
| Niacinamide | 0,1-0,90 |
| Phytospingosine HCl | 0,01-0,1 |
| 1,2-Decanediol | 0,01-3 |
| *Salix alba* (Willow) Bark Extract | 0,01-0,9 |
| PEG-40 Hydrogenated Castor Oil | 0-1,00 |
| Polyquaternium-10 | 0-1 |
| Sodium Cocoamphoacetate | 1-6,00 |
| Disodium Laureth Sulfosuccinate | 1-8,00 |
| Methylpropanediol (80%), Caprylyl Glycol (15%), Phenylpropanol (5%) (Dermosoft OMP) | 1-2,50 |
| Lactic Acid | 0,01-0,90 |
| Demin. H₂O | ad 100,00 |

### Example 9

**Suncream**

| **INCI NAME** | **% (w/w)** |
|---|---|
| | |
| Avobenzone | 0-5 |
| Glycerol | 0-5,00 |
| Panthenol | 0-1,00 |
| Disodium EDTA | 0,10 |
| Glyceryl Stearate, PEG-100 Stearate (50%:50%) (Arlacel 165V P) | 1-5,00 |
| 4-Methylbenzylidene Camphor | 1-4,00 |
| Citrus Aurantius Amara | 0-1 |
| Xanthan Gum | 0-1 |
| Ascorbyl Tetraisopalmitate | 0,20 |
| Polyacrylamide (45%), C13-14 Isoparaffin (25%), Laureth-7 (8%), Aqua (22%) (Sepigel 305) | 0-2,00 |
| Methylene Bis-Benzotriazolyl Tetramethylbutylphenol (46-55%), Aqua (33,9-47,7%), Decyl Glucoside (6-10%), Propylene Glycol 0,2-0,6%), Xanthan Gum (0,1-0,5%) (Tinosorb M) | 0-10,00 |
| Ethylhexyl Methoxycinnamate | 0-8,00 |
| Niacinamide | 0-1 |
| Magnesium Aluminum Silicate | 0-0,50 |
| Dicaprylyl Carbonate | 1-5,00 |
| 1,2-Hexanediol (and) Caprylyl Glycol (50%:50%) (Symdiol 68) | 0,50 |
| Soy Isoflavones | 0,30 |
| Ethylhexyl Triazone | 0-2,00 |
| Titanium Dioxide (75%),Silica (10%), Dimethicone (15%) (Parsol TX) | 0-5 |
| 1,2-Decanediol | 0,30 |
| *Salix alba* (Willow) Bark Extract | 0,30 |
| Tocopheryl Acetate | 0,50 |
| Butylated hydroxytoluene | 0-0,05 |
| Hydroxyacetophenone | 0-0,50 |
| Demin. H₂O | ad 100,00 |

### Example 10

**Suncream 2**

| **INCI NAME** | **% (w/w)** |
|---|---|
| | |
| Avobenzone | 0-5 |
| Glycerol | 0-5,00 |
| Panthenol | 0-1,00 |
| Disodium EDTA | 0,10 |
| Glyceryl Stearate, PEG-100 Stearate (50%:50%) (Arlacel 165V P) | 1-5,00 |
| 4-Methylbenzylidene Camphor | 1-4,00 |
| Citrus Aurantius Amara | 0-1 |
| Xanthan Gum | 0-1 |
| Ascorbyl Tetraisopalmitate | 0,20 |
| Polyacrylamide (45%), C13-14 Isoparaffin (25%), Laureth-7 (8%), Aqua (22%) (Sepigel 305) | 0-2,00 |
| Aqua (37-46%), Tris-Biphenyl Triazine (47-53%), decyl glucoside (6,5-8,5%), disodium phosphate (0.2%-0.6%), butylene glycol (0.2%-0.6%), xanthan gum (0.1%-0.3%) (Tinosorb A2B) | 0-10,00 |
| Ethylhexyl Methoxycinnamate | 0-8,00 |
| Niacinamide | 0-1 |
| Magnesium Aluminum Silicate | 0-0,50 |
| Dicaprylyl Carbonate | 1-5,00 |
| 1,2-Hexanediol (and) Caprylyl Glycol (50%:50%) (Symdiol 68) | 0,50 |
| Soy Isoflavones | 0,30 |
| Ethylhexyl Triazone | 0-2,00 |
| Titanium Dioxide (75%), Silica (10%), Dimethicone (15%) (Parsol TX) | 0-5 |
| 1,2-Decanediol | 0,30 |
| *Salix alba* (Willow) Bark Extract | 0,30 |
| Tocopheryl Acetate | 0,50 |
| Butylated hydroxytoluene | 0-0,05 |
| Hydroxyacetophenone | 0-0,50 |
| Demin. H₂O | ad 100,00 |

## Claims

1. A composition for use in the antibacterial and/or anti-inflammatory treatment of acne comprising 1, 2-decanediol and *Salix alba* bark extract wherein the Salix alba bark extract comprises a total concentration of salicylic derivatives expressed as salicin of not less than 20 % (w/w), and wherein the ratio between Salix alba bark extract and 1, 2-decanediol is 1:1.

2. The composition for use according to claim 1, **characterized in that** the proportion of 1, 2-decanediol is in the range between 0.001 % (w/w)and 10 % (w/w), in particular in the range between 0.01 % (w/w)and 5% (w/w), based on the total mass of the composition.

3. The composition for use according to any one of the preceding claims, **characterized in that** the proportion of *Salix alba* bark extract is in the range between 0.001 % (w/w) and 10 % (w/w), preferably between 0.01 % (w/w) and 5 % (w/w), based on the total mass of the composition.

4. The composition for use according to any one of the preceding claims wherein the composition is in the form of a surfactant system, water-in-oil emulsion, oil-in-water emulsion, serum, gel, oil, aqueous solution, hydroalcoholic solution , anhydrous sticks, compact founding powder, lotion, milk, suncream, after sun product or camouflage product.

5. The composition for use according to any one of the preceding claims additionally comprising customary auxiliaries and additives.

6. The composition for use according to any of claims 1 to 5, characterized that it is a suncream comprising 0.3% (w/w) *Salix alba* bark extract, 0.3% (w/w) 1, 2-decanediol, up to 10% (w/w) of a mixture of Methylene Bis-Benzotriazolyl Tetramethylbutylphenol (46-55%)/Aqua (33,9-47,7%)/Decyl Glucoside (6-10%)/Propylene Glycol 0,2-0,6%)/Xanthan Gum (0,1-0,5%), up to 0.5% (w/w) Magnesium Aluminum Silicate, up to 5% (w/w) of a mixture of Titanium Dioxide (75%)/Silica (10%)/Dimethicone (15%), up to 8% (w/w) Ethylhexyl Methoxycinnamate, up to 5% (w/w) Dicaprylyl Carbonate, up to 5% (w/w) Avobenzone, up to 2% (w/w) Ethylhexyl Triazone.

7. The composition for use according to any of claims 1 to 5, characterized that it is a suncream comprising 0.3% (w/w) *Salix alba* bark extract, 0.3% (w/w) 1, 2-decanediol, up to 10% (w/w) of a mixture of Aqua (37-46%)/Tris-Biphenyl Triazine (47-53%)/decyl glucoside (6,5-8,5%)/disodium phosphate (0.2%-0.6%)/butylene glycol (0.2%-0.6%)/xanthan gum (0.1%-0.3%), up to 0.5% (w/w) Magnesium Aluminum Silicate, up to 5% (w/w) of a mixture of Titanium Dioxide (75%)/Silica (10%)/Dimethicone (15%)/up to 8% (w/w) Ethylhexyl Methoxycinnamate, up to 5% (w/w) Dicaprylyl Carbonate, up to 5% (w/w) Avobenzone, up to 2% (w/w) Ethylhexyl Triazone.

8. A composition for use in the antibacterial and/or anti-inflammatory treatment of acne comprising 1, 2-decanediol and *Salix alba* bark extract wherein the Salix alba bark extract comprises a total concentration of salicylic derivatives expressed as salicin of not less than 20 % (w/w),, characterized that it is a detergent comprising 0.01 to 0.9% (w/w) *Salix alba* bark extract, 0.01 to 3% (w/w) 1, 2-decanediol, 0.01 to 0.1% (w/w) phytosphingosine HCI, up to 6% (w/w) sodium cocoamphoacetate, up to 8% (w/w) disodiumlaureth sulfosuccinate, up to 1% (w/w) polyquaternium-10.

9. A composition for use in the antibacterial and/or anti-inflammatory treatment of acne comprising 1, 2-decanediol and *Salix alba* bark extract wherein the Salix alba bark extract comprises a total concentration of salicylic derivatives expressed as salicin of not less than 20 % (w/w),, characterized that it is a moisturizing cream comprising 0.01 to 0.3% (w/w) *Salix alba* bark extract, up to 0.9% (w/w) 1, 2-decanediol, 0.2 to 0.9% (w/w) sodium hyaluronate, 1 to 5% (w/w) dicaprylyl ether, up to 2% (w/w) aluminium starch octenylsuccinate, up to 0.5% (w/w) sodium phytate.

10. A composition for use in the antibacterial and/or anti-inflammatory treatment of acne comprising 1, 2-decanediol and *Salix alba* bark extract wherein the Salix alba bark extract comprises a total concentration of salicylic derivatives expressed as salicin of not less than 20 % (w/w),, characterized that it is a CC cream comprising 0.5% (w/w) *Salix alba* bark extract, 1% (w/w) 1, 2-decanediol, up to 2% (w/w) of a mixture of Methylpropanediol (80%)/Caprylyl Glycol (15%)/Phenylpropanol (5%), up to 5% (w/w) titanium dioxide, up to 2% (w/w) of a mixture of Mica (44-54%)/Iron Oxides (36-50%)/Titanium Dioxide (2-8%), up to 0.5% (w/w) magnesium sulfate heptahydrate.

11. A composition for use in the antibacterial and/or anti-inflammatory treatment of acne comprising 1, 2-decanediol and *Salix alba* bark extract wherein the Salix alba bark extract comprises a total concentration of salicylic derivatives expressed as salicin of not less than 20 % (w/w),, characterized that it is a liquid detergent comprising 0.01% (w/w) *Salix alba* bark extract, 1% (w/w) 1, 2-decanediol, up to 1% (w/w) of hydroxypropyl guar hydroxypropyltrimonium chloride, up to 10% (w/w) sodium cocoamphoacetate, up to 5% of a mixture of Methylpropanediol (80%)/Caprylyl Glycol (15%)/Phenylpropanol (5%).

12. The composition for use according to any of claims 1 to 5, characterized that it is a gel comprising 0.5% (w/w) *Salix alba* bark extract, 0.5% (w/w) 1, 2-decanediol, up to 2% (w/w) caprylyl glycol, up to 3% (w/w) polyacrylate crosspolymer-6, up to 1% (w/w) niacinamide.

13. A composition for use in the antibacterial and/or anti-inflammatory treatment of acne comprising 1, 2-decanediol and *Salix alba* bark extract wherein the Salix alba bark extract comprises a total concentration of salicylic derivatives expressed as salicin of not less than 20 % (w/w),, characterized that it is a serum comprising 0.3% (w/w) *Salix alba* bark extract, 1% (w/w) 1, 2-decanediol, up to 4% (w/w) of a mixture of Polyglyceryl-6 Stearate (85-98%)/Polyglyceryl-6 Behenate (2-15%), up to 5% (w/w) caprylyl glycol, up to 2% (w/w) salicylic acid, up to 2% (w/w) aluminium starch octenylsuccinate, up to 2% (w/w) dipotassium glycirrhizinate.

## Patentansprüche

1. Zusammensetzung zur Verwendung bei der antibakteriellen und/oder entzündungshemmenden Behandlung von Akne, umfassend 1,2-Decandiol und Salix alba-Rindenextrakt, wobei der Salix alba-Rindenextrakt eine Gesamtkonzentration von als Salicin exprimierten Salicylderivaten von nicht weniger als 20 % (w/w) umfasst, und wobei das Verhältnis zwischen dem Salix alba-Rindenextrakt und 1,2-Decandiol 1:1 beträgt.

2. Zusammensetzung zur Verwendung nach Anspruch 1, **dadurch gekennzeichnet, dass** der Anteil von 1,2-Decandiol im Bereich von zwischen 0,001 % (w/w) und 10 % (w/w), insbesondere im Bereich von zwischen 0,01 % (w/w) und 5 % (w/w), basierend auf der Gesamtmasse der Zusammensetzung liegt.

3. Zusammensetzung zur Verwendung nach einem der vorstehenden Ansprüche, **dadurch gekennzeichnet, dass** der Anteil des Salix alba-Rindenextrakts im Bereich von zwischen 0,001 % (w/w) und 10 % (w/w), bevorzugt zwischen 0,01 % (w/w) und 5 % (w/w), basierend auf der Gesamtmasse der Zusammensetzung liegt.

4. Zusammensetzung zur Verwendung nach einem der vorstehenden Ansprüche, wobei die Zusammensetzung die Form eines Tensidsystems, einer Wasser-in-Öl-Emulsion, Öl-in-Wasser-Emulsion, eines Serums, Gels, Öls, einer wässrigen Lösung, hydroalkoholischen Lösung, von wasserfreien Stäben, kompakten Gießpulvers, einer Lotion, Milch, Sonnencreme, eines After-Sun-Produkts oder Camouflageprodukts aufweist.

5. Zusammensetzung zur Verwendung nach einem der vorstehenden Ansprüche, die des Weiteren handelsübliche Hilfs- und Zusatzstoffe umfasst.

6. Zusammensetzung zur Verwendung nach einem der Ansprüche 1 bis 5, **dadurch gekennzeichnet, dass** sie eine Sonnencreme ist, die 0,3 % (w/w) Salix alba-Rindenextrakt, 0,3 % (w/w) 1,2-Decandiol, bis zu 10 % (w/w) einer Mischung aus Methylen Bis-Benzotriazolyl Tetramethylbutylphenol (46-55 %)/Aqua (33,9-47,7 %)/Decylglucosid (6-10 %)/Propylenglykol (0,2-0,6 %)/Xanthangummi (0,1-0,5 %), bis zu 0,5 % (w/w) Magnesiumaluminiumsilikat, bis zu 5 % (w/w) einer Mischung aus Titandioxid (75 %)/Silizimdioxid (10 %)/Dimethicon (15 %), bis zu 8 % (w/w) Ethylhexylmethoxycinnamat, bis zu 5 % (w/w) Dicaprylylcarbonat, bis zu 5 % (w/w) Avobenzon, bis zu 2 % (w/w) Ethylhexyltriazon umfasst.

7. Zusammensetzung zur Verwendung nach einem der Ansprüche 1 bis 5, **dadurch gekennzeichnet, dass** sie eine Sonnencreme ist, die 0,3 % (w/w) Salix alba-Rindenextrakt, 0,3 % (w/w) 1,2-Decandiol, bis zu 10 % (w/w) einer Mischung aus Aquamarin (37-46 %)/Tris-Biphenyltriazin (47-53 %)/Decylglucosid (6,5-8,5 %)/Dinatriumphosphat (0,2 %-0,6 %)/Butylenglykol (0,2 %-0,6 %)/Xanthangummi (0,1 %-0,3 %), bis zu 0,5 % (w/w) Magnesiumaluminiumsilikat, bis zu 5 % (w/w) einer Mischung aus Titandioxid (75 %)/Silizimdioxid (10 %)/Dimethicon (15 %)/bis zu 8 % (w/w) Ethylhexylmethoxycinnamat, bis zu 5 % (w/w) Dicaprylylcarbonat, bis zu 5 % (w/w) Avobenzon, bis zu 2 % (w/w) Ethylhexyltriazon umfasst.

8. Zusammensetzung zur Verwendung bei der antibakteriellen und/oder entzündungshemmenden Behandlung von Akne, umfassend 1,2-Decandiol und Salix alba-Rindenextrakt, wobei der Salix alba-Rindenextrakt eine Gesamtkonzentration von als Salicin exprimierten Salicylderivaten von nicht weniger als 20 % (w/w) umfasst, **dadurch gekennzeichnet, dass** es ein Detergens ist, das 0,01 bis 0,9 % (w/w) Salix alba-Rindenextrakt, 0,01 bis 3 % (w/w) 1,2-Decandiol, 0,01 bis 0,1 % (w/w) Phytosphingosin HCI, bis zu 6 % (w/w) Natrium-Cocoamphoacetat, bis zu 8 % (w/w) Dinatriumlaurethsulfosuccinat, bis zu 1% (w/w) Polyquaternium-10 umfasst.

9. Zusammensetzung zur Verwendung bei der antibakteriellen und/oder entzündungshemmenden Behandlung von Akne, umfassend 1,2-Decandiol und Salix alba-Rindenextrakt, wobei der Salix alba-Rindenextrakt eine Gesamtkonzentration von als Salicin exprimierten Salicylderivaten von nicht weniger als 20 % (w/w) umfasst, **dadurch gekennzeichnet, dass** sie eine Feuchtigkeitscreme ist, die 0,01 bis 0,3 % (w/w) Salix alba-Rindenextrakt, bis zu 0,9 % (w/w) 1,2-Decandiol, 0,2 bis 0,9 % (w/w) Hyaluronsäure, 1 bis 5 % (w/w) Dicaprylylether, bis zu 2 % (w/w) Stärkealuminiumoctenylsuccinat, bis zu 0,5 % (w/w) Natriumphytat umfasst.

10. Zusammensetzung zur Verwendung bei der antibakteriellen und/oder entzündungshemmenden Behandlung von Akne, umfassend 1,2-Decandiol und Salix alba-Rindenextrakt, wobei der Salix alba-Rindenextrakt eine Gesamtkonzentration von als Salicin exprimierten Salicylderivaten von nicht weniger als 20 % (w/w) umfasst, **dadurch gekennzeichnet, dass** sie eine CC-Creme ist, die 0,5 % (w/w) Salix alba-Rindenextrakt, 1% (w/w) 1,2-Decandiol, bis zu 2 % (w/w) einer Mischung aus Methylpropandiol (80 %)/Caprylylglykol (15 %)/Phenylpropanol (5 %), bis zu 5 % (w/w) Titandioxid, bis zu 2 % (w/w) einer Mischung aus Glimmer (44-54 %)/Eisenoxiden (36-50 %)/Titandioxid (2-8 %), bis zu 0,5 % (w/w) Magnesiumsulfatheptahydrat umfasst.

11. Zusammensetzung zur Verwendung bei der antibakteriellen und/oder entzündungshemmenden Behandlung von Akne, umfassend 1,2-Decandiol und Salix alba-Rindenextrakt, wobei der Salix alba-Rindenextrakt eine Gesamtkonzentration von als Salicin exprimierten Salicylderivaten von nicht weniger als 20 % (w/w) umfasst, **dadurch gekennzeichnet, dass** sie ein Flüssigwaschmittel ist, das 0,01 % (w/w) Salix alba-Rindenextrakt, 1 % (w/w) 1,2-Decandiol, bis zu 1 % (w/w) von Hydroxypropyl-GuarHydroxypropyltrimoniumchlorid, bis zu 10 % (w/w) Natrium-Cocoamphoacetat, bis zu 5 % einer Mischung aus Methylpropandiol (80 %)/Caprylylglykol (15 %)/Phenylpropanol (5 %) umfasst.

12. Zusammensetzung zur Verwendung nach einem der Ansprüche 1 bis 5 **dadurch gekennzeichnet, dass** sie ein Gel ist, das 0,5 % (w/w) Salix alba-Rindenextrakt, 0,5 % (w/w) 1,2-Decandiol, bis zu 2 % (w/w) Capryiylglykol, bis zu 3 % (w/w) Polyacrylat-Crosspolymer-6, bis zu 1 % (w/w) Niacinamid umfasst.

13. Zusammensetzung zur Verwendung bei der antibakteriellen und/oder entzündungshemmenden Behandlung von Akne, umfassend 1,2-Decandiol und Salix alba-Rindenextrakt, wobei der Salix alba-Rindenextrakt eine Gesamtkonzentration von als Salicin exprimierten Salicylderivaten von nicht weniger als 20 % (w/w) umfasst, **dadurch gekennzeichnet, dass** sie ein Serum ist, das 0,3 % (w/w) Salix alba-Rindenextrakt, 1 % (w/w) 1,2-Decandiol, bis zu 4 % (w/w) einer Mischung aus Polyglyceryl-6-Stearat (85-98 %)/Polyglyceryl-6-Behenat (2-15 %), bis zu 5 % (w/w) Caprylylglykol, bis zu 2 % (w/w) Salicylsäure, bis zu 2 % (w/w) Stärkealuminiumoctenylsuccinat, bis zu 2 % (w/w) Dikaliumglycirrhizinat umfasst.

## Revendications

1. Composition destinée à une utilisation dans le traitement antibactérien et/ou anti-inflammatoire de l'acné, comprenant du 1,2-décanediol et de l'extrait d'écorce de Salix alba, l'extrait d'écorce de Salix alba comprenant une concentration totale de dérivés salicyliques exprimée en salicine non inférieure à 20 % (p/p), et le rapport entre l'extrait d'écorce de Salix alba et le 1,2-décanediol étant de 1:1.

2. Composition destinée à une utilisation selon la revendication 1, **caractérisée en ce que** la proportion de 1,2-décanediol se situe dans la plage comprise entre 0,001 % (p/p) et 10 % (p/p), en particulier dans la plage comprise entre 0,01 % (p/p) et 5 % (p/p), par rapport à la masse totale de la composition.

3. Composition destinée à une utilisation selon l'une quelconque des revendications précédentes, **caractérisée en ce que** la proportion d'extrait d'écorce de Salix alba se situe dans la plage comprise entre 0,001 % (p/p) et 10 % (p/p), de préférence entre 0,01 % (p/p) et 5 % (p/p), par rapport à la masse totale de la composition.

4. Composition destinée à une utilisation selon l'une quelconque des revendications précédentes, la composition se présentant sous la forme d'un système tensioactif, d'une émulsion eau dans l'huile, d'une émulsion huile dans l'eau, d'un sérum, d'un gel, d'une huile, d'une solution aqueuse, d'une solution hydroalcoolique, de bâtonnets anhydres, d'une poudre compacte, d'une lotion, d'un lait, d'une crème solaire, d'un produit après-soleil ou d'un produit correcteur.

5. Composition destinée à une utilisation selon l'une quelconque des revendications précédentes, comprenant également des adjuvants et additifs classiques.

6. Composition destinée à une utilisation selon l'une quelconque des revendications 1 à 5, **caractérisée en ce qu'**il s'agit d'une crème solaire comprenant 0,3 % (p/p) d'extrait d'écorce de Salix alba, 0,3 % (p/p) de 1,2-décanediol, jusqu'à 10 % (p/p) d'un mélange de méthylène bis-benzotriazolyl tétraméthylbutylphénol (46 à 55 %)/eau (33,9 à 47,7 %)/décylglucoside (6 à 10 %)/propylène glycol (0,2 à 0,6 %)/gomme de xanthane (0,1 à 0,5 %), jusqu'à 0,5 % (p/p) de silicate de magnésium et d'aluminium, jusqu'à 5 % (p/p) d'un mélange de dioxyde de titane (75 %)/silice (10 %)/diméthicone (15 %), jusqu'à 8 % (w/w) de méthoxycinnamate d'éthylhexyle, jusqu'à 5 % (p/p) de carbonate de dicaprylyle, jusqu'à 5 % (p/p) d'avobenzone, jusqu'à 2 % (p/p) d'éthylhexyltriazone.

7. Composition destinée à une utilisation selon l'une quelconque des revendications 1 à 5, **caractérisée en ce qu'**il s'agit d'une crème solaire comprenant 0,3 % (p/p) d'extrait d'écorce de Salix alba, 0,3 % (p/p) de 1,2-décanediol, jusqu'à 10 % (p/p) d'un mélange d'eau (37 à 46 %)/tris-biphényl triazine (47 à 53 %)/décylglucoside (6,5 à 8,5 %)/phosphate disodique (0,2 % à 0,6 %)/butylène glycol (0,2 % à 0,6 %)/gomme de xanthane (0,1 % à 0,3 %), jusqu'à 0,5 % (p/p) de silicate de magnésium et d'aluminium, jusqu'à 5 % (p/p) d'un mélange de dioxyde de titane (75 %)/silice (10 %)/diméthicone (15 %), jusqu'à 8 % (w/w) de méthoxycinnamate d'éthylhexyle, jusqu'à 5 % (p/p) de carbonate de dicaprylyle, jusqu'à 5 % (p/p) d'avobenzone, jusqu'à 2 % (p/p) d'éthylhexyltriazone.

8. Composition destinée à une utilisation dans le traitement antibactérien et/ou anti-inflammatoire de l'acné, comprenant du 1,2-décanediol et de l'extrait d'écorce de Salix alba, l'extrait d'écorce de Salix alba comprenant une concentration totale de dérivés salicyliques exprimés en salicine non inférieure à 20 % (p/p), **caractérisée en ce qu'**il s'agit d'un détergent comprenant 0,01 à 0,9 % (p/p) d'extrait d'écorce de Salix alba, 0,01 à 3 % (p/p) de 1,2-décanediol, 0,01 à 0,1 % (p/p) de chlorhydrate de phytosphingosine, jusqu'à 6 % (p/p) de cocoamphoacétate de sodium, jusqu'à 8 % (p/p) de sulfosuccinate de disodium laureth, jusqu'à 1 % (p/p) de polyquaternium-10.

9. Composition destinée à une utilisation dans le traitement antibactérien et/ou anti-inflammatoire de l'acné, comprenant du 1,2-décanediol et de l'extrait d'écorce de Salix alba, l'extrait d'écorce de Salix alba comprenant une concentration totale de dérivés salicyliques exprimés en salicine non inférieure à 20 % (p/p), **caractérisée en ce qu'**il s'agit d'une crème hydratante comprenant 0,01 à 0,3 % (p/p) d'extrait d'écorce de Salix alba, jusqu'à 0,9 % (p/p) de 1,2-décanediol, 0,2 à 0,9 % (w/w) d'hyaluronate de sodium, 1 à 5 % (p/p) d'éther dicaprylylique, jusqu'à 2 % (p/p) d'octénylsuccinate d'amidon et d'aluminium, jusqu'à 0,5 % (p/p) de phytate de sodium.

10. Composition destinée à une utilisation dans le traitement antibactérien et/ou anti-inflammatoire de l'acné, comprenant du 1,2-décanediol et de l'extrait d'écorce de Salix alba, l'extrait d'écorce de Salix alba comprenant une concentration totale de dérivés salicyliques exprimés en salicine non inférieure à 20 % (p/p), **caractérisée en ce qu'**il s'agit d'une CC crème comprenant 0,5 % (p/p) d'extrait d'écorce de Salix alba, 1 % (p/p) de 1,2-décanediol, jusqu'à 2 % (p/p) d'un mélange de méthylpropanediol (80 %)/caprylylglycol (15 %)/phénylpropanol (5 %), jusqu'à 5 % (p/p) de dioxyde de titane, jusqu'à 2 % (p/p) d'un mélange de mica (44 à 54 %)/oxydes de fer (36 à 50 %)/dioxyde de titane (2 à 8 %), jusqu'à 0,5 % (w/w) de sulfate de magnésium heptahydraté.

11. Composition destinée à une utilisation dans le traitement antibactérien et/ou anti-inflammatoire de l'acné, comprenant du 1,2-décanediol et de l'extrait d'écorce de Salix alba, l'extrait d'écorce de Salix alba comprenant une concentration totale de dérivés salicyliques exprimés en salicine non inférieure à 20 % (p/p), **caractérisée en ce qu'**il s'agit d'un détergent liquide comprenant 0,01 % (p/p) d'extrait d'écorce de Salix alba, 1 % (p/p) de 1,2-décanediol, jusqu'à 1 % (p/p) de chlorure d'hydroxypropyl guar hydroxypropyltrimonium, jusqu'à 10 % (p/p) de cocamphoacétate de sodium, jusqu'à 5 % d'un mélange de méthylpropanediol (80 %)/caprylylglycol (15 %)/phénylpropanol (5 %) .

12. Composition destinée à une utilisation selon l'une quelconque des revendications 1 à 5, **caractérisée en ce qu'**il s'agit d'un gel comprenant 0,5 % (p/p) d'extrait d'écorce de Salix alba, 0,5 % (p/p) de 1,2-décanediol, jusqu'à 2 % (p/p) de caprylylglycol, jusqu'à 3 % (p/p) de polymère croisé de polyacrylate 6, jusqu'à 1 % (p/p) de niacinamide.

13. Composition destinée à une utilisation dans le traitement antibactérien et/ou anti-inflammatoire de l'acné, comprenant du 1,2-décanediol et de l'extrait d'écorce de Salix alba, l'extrait d'écorce de Salix alba comprenant une concentration totale de dérivés salicyliques exprimés en salicine non inférieure à 20 % (p/p), **caractérisée en ce qu'**il s'agit d'un sérum comprenant 0,3 % (p/p) d'extrait d'écorce de Salix alba, 1 % (p/p) de 1,2-décanediol, jusqu'à 4 % (p/p) d'un mélange de stéarate de polyglycéryle 6 (85 à 98 %)/béhénate de polyglycéryle 6 (2 à 15 %), jusqu'à 5 % (p/p) de caprylylglycol, jusqu'à 2 % (p/p) d'acide salicylique, jusqu'à 2 % (p/p) d'octénylsuccinate d'amidon et d'aluminium, jusqu'à 2 % (p/p) de glycyrrhizinate de dipotassium.
